# EUROPEAN PATENT APPLICATION

(11) **EP 2 256 109 A1**
(43) Date of publication of application: **01.12.2010**
(21) Application number: 09704252.7
(22) Date of filing: 23.01.2009
(51) Int. Cl.: C07D 295/12, A61K 31/495, A61P 3/10, A61P 9/10, A61P 17/02, A61P 25/28, A61P 35/00, A61P 43/00

(54) **APOPTOSIS INHIBITOR**

(30) Priority: 25.01.2008 JP 2008014835
(71) Applicant: Nihon University, Tokyo 102-8275 (JP)
(72) Inventor: ISHIGE, Kumiko, Tokyo 102-8275 (JP); ITO, Yoshihisa, Tokyo 102-8275 (JP); HIRABAYASHI, Mioko, Tokyo 102-8275 (JP)
(74) Representative: Blodig, Wolfgang
(86) International application number: PCT/JP2009/000268
(87) International publication number: WO 2009/093471

(57) **Abstract**

It is directed to provide a novel apoptosis inhibitor. Specifically directed is an apoptosis inhibitor containing a biphenylcarboxamide compound represented by the following formula (1) as an active ingredient, wherein R¹ represents a hydrogen atom or a lower alkoxy group; R² represents a hydrogen atom or a lower alkanoyl group; and n represents a number of 2 to 5.

## Description

### [Technical Field]

The present invention relates to a pharmaceutical inhibiting apoptosis induced by oxidative stress.

### [Background Art]

Reactive oxygen is known to oxidatively denature lipids, proteins, sugars, nucleic acids, and the like *in vivo* and impair cellular functions. *In-vivo* overproduction of reactive oxygen is thought to increase oxidative stress *in vivo*, resulting in the onset of diseases such as arteriosclerosis, myocardial infarction, diabetes, and cancer. Moreover, nerve cell death in brain tissues is considered to be also caused by an excess of NO produced by activated microglia.

Thus, in-vivo cell death (apoptosis) caused by oxidative stress *in vivo* is deeply involved in the development and progression of various diseases. Accordingly, ingredients that inhibit the apoptosis have been demanded.

On the other hand, biphenylcarboxamide compounds typified by 4'-acetyl-N-[4-[4-(2-methoxyphenyl)-l-piperazinyl]butyl]-[1,1'-biphenyl]-4-carboxamide (GR103691) are commercially available as dopamine D3 receptor antagonists (Non Patent Documents 1 and 2).
[Non Patent Document 1] Eur. J. Pharmacol. 1996 Dec. 30; 318 (2-3); 283-293
[Non Patent Document 2] J. Pharmacol. Exp. Ther. 1998 Oct; 287 (1); 187-197

### [Summary of Invention]

### [Problem to be Solved by the Invention]

An object of the present invention is to provide a novel apoptosis inhibitor.

### [Means for Solving the Problem]

Thus, the present inventors have studied the apoptosis inhibitory effects of various compounds using a hypoxia/reoxygenation-induced cell death model system, which is a model of cell death under oxidative stress, in a cultured cell system. Consequently, the present inventors have found that, unexpectedly, a biphenylcarboxamide compound known as a dopamine D3 receptor antagonist has strong apoptosis inhibitory effect. Based on the findings, the present invention has been completed.

Specifically, the present invention provides an apoptosis inhibitor containing a biphenylcarboxamide compound represented by the following formula (1) as an active ingredient:

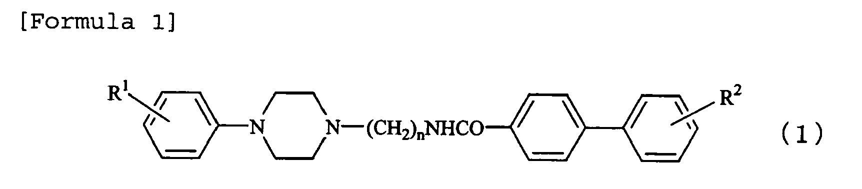

wherein R¹ represents a hydrogen atom or a lower alkoxy group; R² represents a hydrogen atom or a lower alkanoyl group; and n represents a number of 2 to 5.
Moreover, the present invention provides a preventive/therapeutic agent for a disease caused by oxidative stress-induced apoptosis, the preventive/therapeutic agent containing the compound (1) as an active ingredient.
Moreover, the present invention provides a pharmaceutical composition for apoptosis inhibition, containing the compound (1) and a pharmaceutically acceptable carrier.
Moreover, the present invention provides a pharmaceutical composition for prevention/treatment of a disease caused by oxidative stress-induced apoptosis, the pharmaceutical composition containing the compound (1) and a pharmaceutically acceptable carrier.
Moreover, the present invention provides use of the compound (1) for production of an apoptosis inhibitor.
Moreover, the present invention provides use of the compound (1) for production of a preventive/therapeutic agent for a disease caused by oxidative stress-induced apoptosis.
Moreover, the present invention provides a method for inhibiting apoptosis, including administering an effective amount of the compound (1).
Moreover, the present invention provides a method for preventing/treating a disease caused by oxidative stress-induced apoptosis, including administering an effective amount of the compound (1).
Moreover, the present invention provides the compound (1) for apoptosis inhibition.
Furthermore, the present invention provides the compound (1) for prevention/treatment of a disease caused by oxidative stress-induced apoptosis.

### [Advantageous effects of Invention]

A compound of the formula (1) exhibits strong inhibitory effect on oxidative stress-induced apoptosis and is therefore useful as a therapeutic agent for diseases associated with oxidative stress-induced cell death, for example, arteriosclerosis, myocardial infarction, diabetes, cancer, Alzheimer's disease, reperfusion injury, and cutaneous vasculitis.

### [Brief Description of the Drawings]

Figure 1 shows the influence of GR103691 on hypoxia/reoxygenation-induced cell death (LDH release).
Figure 2 shows the influence of nafadotride on hypoxia/reoxygenation-induced cell death.
Figure 3 shows the influence of GR103691 on glutamic acid-induced cell death.
Figure 4 shows the influence of GR103691 on tunicamycin-induced cell death.
Figure 5 shows a PI stain image of hypoxia/reoxygenation-induced cell death.
Figure 6 shows the influence of GR103691 on increase in intracellular reactive oxygen species (ROS) caused by hypoxia-reoxygenation loading.
Figure 7 shows an FJB stain image of the hippocampal CA1 region in a mouse cerebral ischemia/reperfusion model.
Figure 8 shows the proportion of the number of FJB-positive cells to the total number of cells.
Figure 9 shows the proportion of the image of ssDNA-positive cells to the total number of cells in the hippocampal CA1 region in a mouse cerebral ischemia/reperfusion model (Figure 9(a)) and ssDNA stain images (Figure 9(b)).

### [Modes for Carrying out the Invention]

An active ingredient of an apoptosis inhibitor of the present invention is a biphenylcarboxamide compound represented by the formula (1). In the formula (1), a lower alkoxy group represented by R¹ includes alkoxy groups having 1 to 6 carbon atoms, for example, methoxy, ethoxy, propoxy, isopropyloxy, and butoxy groups. A methoxy group is particularly preferable.

In the formula (1), a lower alkanoyl group represented by R² includes alkanoyl groups having 2 to 6 carbon atoms, for example, acetyl, propionyl, and butyryl groups. An acetyl group is particularly preferable.

In the formula (1), n represents a number of 2 to 5 and is preferably 3 to 5, particularly preferably 4.

A compound represented by the formula (1) wherein R¹ is a methoxy group, R² is an acetyl group, and n is 4 is more preferable. Particularly, the GR103691 (4'-acetyl-N-[4-[4-(2-methoxyphenyl)-1-piperazinyl]butyl]-[1,1'-biphenyl]-4-carboxamide) described above is preferable.

The compound of the formula (1) is known per se in the art. For example, GR103691 is commercially available as a dopamine D3 receptor antagonist.

The compound of the formula (1) exhibited strong inhibitory effect on hypoxia/reoxygenation-induced cell death which is oxidative stress-induced cell death, as shown in Examples described later. On the other hand, nafadotride, also known as a dopamine D3 receptor antagonist as with GR103691, differs in chemical structure from the compound of the formula (1) and did not inhibit apoptosis in a similar test system. Moreover, the compound of the formula (1) also inhibited glutamic acid-induced cell death which is one type of oxidative stress-induced cell death and differs in induction mechanism from hypoxia/reoxygenation-induced cell death. Furthermore, the compound of the formula (1) significantly inhibited increase of intracellular reactive oxygen species (ROS) caused by hypoxia-reoxygenation loading. Therefore, owing to the ROS increase inhibitory effect, the compound of the formula (1) presumably imparts inhibitory effect on oxidative stress-induced cell death. Accordingly, the apoptosis inhibitory effect of the present invention is considered as not the effect of a dopamine D3 receptor antagonist but the effect specific to the compound of the formula (1).
Thus, the apoptosis inhibitor of the present invention is useful as a therapeutic agent for various diseases caused by oxidative stress-induced apoptosis, for example, arteriosclerosis, myocardial infarction, cancer, Alzheimer's disease, reperfusion injury (reperfusion injury in brain infarction, etc.), and cutaneous vasculitis.

The pharmaceuticals of the present invention is obtained by appropriately adding one or more pharmaceutically acceptable carriers such as diluents, binders, lubricants, disintegrants, coating agents, emulsifying agents, suspending agents, solvents, stabilizing agents, absorption aids, and ointment bases to the compound of the formula (1) and preparing dosage forms for oral administration, injection, intrarectal administration, external use, or the like according to a ordinary method.

The preparations for oral administration are preferably granules, tablets, coated tablets, capsules, pills, liquids, emulsions, suspensions, and the like; the preparations for injection are preferably preparations for intravenous injection, intramuscular injection, hypodermic injection, drip infusion, and the like; and the preparations for intrarectal administration are preferably soft capsule type suppositories and the like.
The pharmaceutical of the present invention can be administered in the forms of such preparations to mammals including humans.
The pharmaceutical of the present invention is preferably administered approximately 1 to 500 mg/kg per day in terms of the amount of the compound of the formula (1) as a single administration or 2 to 4-divided administrations.

### [Examples]

Hereinafter, the present invention will be described more specifically with reference to Examples. However, the present invention is not limited to these Examples by any means.

### Example 1

### (Method)

### (1) Cell culture

HT22 cells were cultured in DMEM containing 10% FBS in a 10% CO₂ incubator at 37°C.

### (2) Hypoxia-reoxygenation loading

Hypoxia loading was performed by using an oxygen absorber/CO₂ generator AnaeroPack Kenki For Cell^{™}, and reoxygenation was performed by restoring the condition to normoxia.

### (3) Lactate dehydrogenase (LDH) assay

LDH assay was conducted using a supernatant of a medium of cells treated as intended as a sample and a measurement kit (LDH cytotoxic test).
Specifically, the cells were cultured in a 96-well assay plate and treated as intended. Then, 50 µL of only the medium was collected and transferred to a newly prepared plate. A coloring reagent was added to each well. After incubation for 20 minutes, 100 µL of a reaction stop solution was added thereto, and the absorbance at 570 nm was measured using a microplate reader. The results are indicated relative to the absorbance of control cells defined as 100%. In this context, LDH is an enzyme released from dead cells, and increase of LDH level in the medium indicates that cell death occurs.

### (4) MTT assay

MTT assay was conducted according to a ordinary method by using cells treated as intended as a sample.
Specifically, the cells were cultured in a 96-well assay plate and treated as intended. Then, 10 µL of an MTT solution was added to each well, followed by incubation for 4 hours. 100 µL of a reaction stop solution was added thereto. The plate was left overnight, and the absorbance (measurement wavelength: 570 nm, control wavelength: 655 nm) was then measured using a microplate reader. The results are indicated relative to the absorbance of control cells defined as 100%. In this context, the MTT value is a value that indicates mitochondrial reductive capacity. This value correlates with cell viability in many cases and is therefore used as an index of cell viability. It has been confirmed as to the glutamic acid-induced cell death of HT22 cells that the results of MTT assay correlate with cell viability.

### (Results)

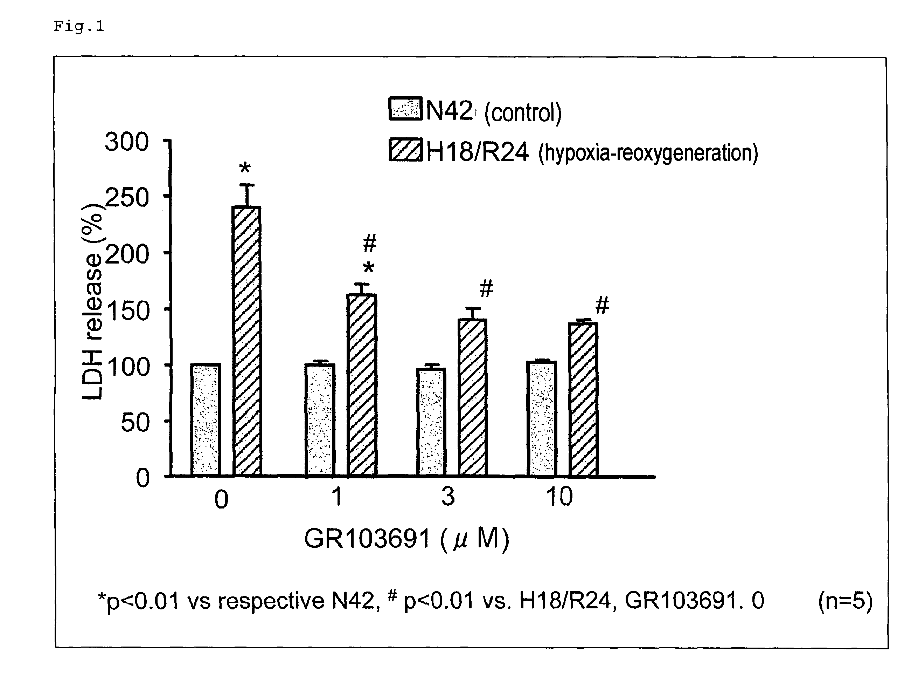

### (1) The influence of GR103691 on hypoxia/reoxygenation-induced cell death is shown in Figure 1.

LDH release was measured after hypoxia for 18 hours (H18) and subsequent reoxygenation for 24 hours (R24). Cells placed under normoxia for 42 (18+24) hours (N42) were used as a control. GR103691 was added immediately before reoxygenation (or after 18-hour culture to the control cells).

Significant increase of LDH release after hypoxia-reoxygenation (H18R24) compared with the control (N42), i.e., cell death, was observed. The addition of GR103691 immediately before reoxygenation reduced the LDH release of the H18/R24-treated cells in a concentration-dependent manner, and the reduction due to the addition of 1 µM or higher GR103691 was significant. By contrast, the LDH release of the control cells was not influenced by GR103691. Moreover, increase of LDH was not observed after only hypoxia loading for 18 hours. LDH increased along with reoxygenation and significantly increased after reoxygeneration for 18 hours (H18/R18).

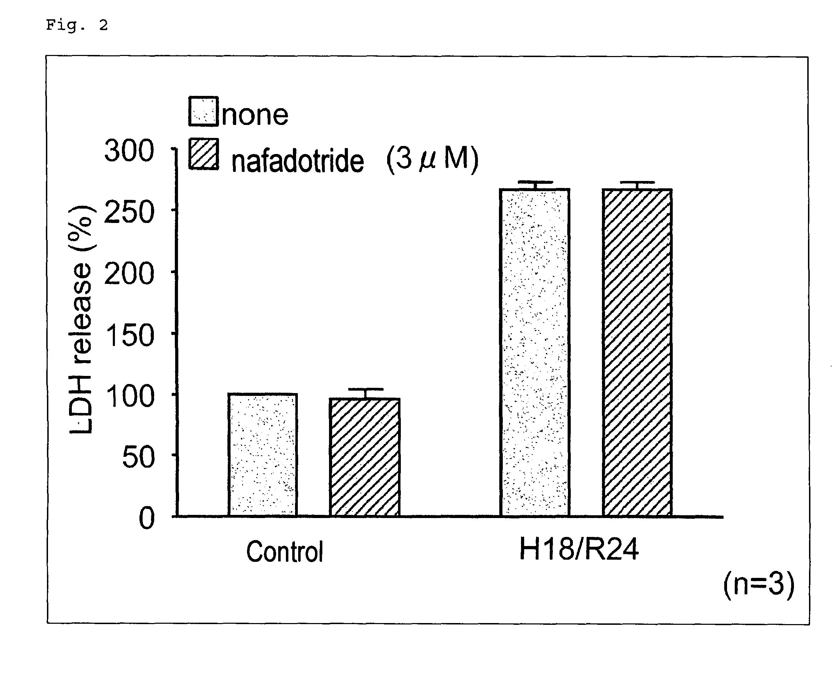

### (2) The influence of nafadotride on hypoxia/reoxygenation-induced cell death is shown in Figure 2.

LDH release was measured after hypoxia for 18 hours (H18) and subsequent reoxygenation for 24 hours (R24). Cells placed under normoxia for 42 (18+24) hours (N42) were used as a control. Nafadotride was added immediately before reoxygenation (or after 18-hour culture for the control cells).

Since GR103691 is currently commercially available as a D3 antagonist, the influence of nafadotride put on the market as a D3 antagonist was studied in order to examine whether or not the effect of Figure 1 is common to D3 antagonists. As in GR103691, nafadotride was added immediately before reoxygenation and however, did not influence increase of LDH release caused by H18/R24. In this test, nafadotride was used at a concentration of 3 µM, at which GR103691 exhibited almost the maximum effect.

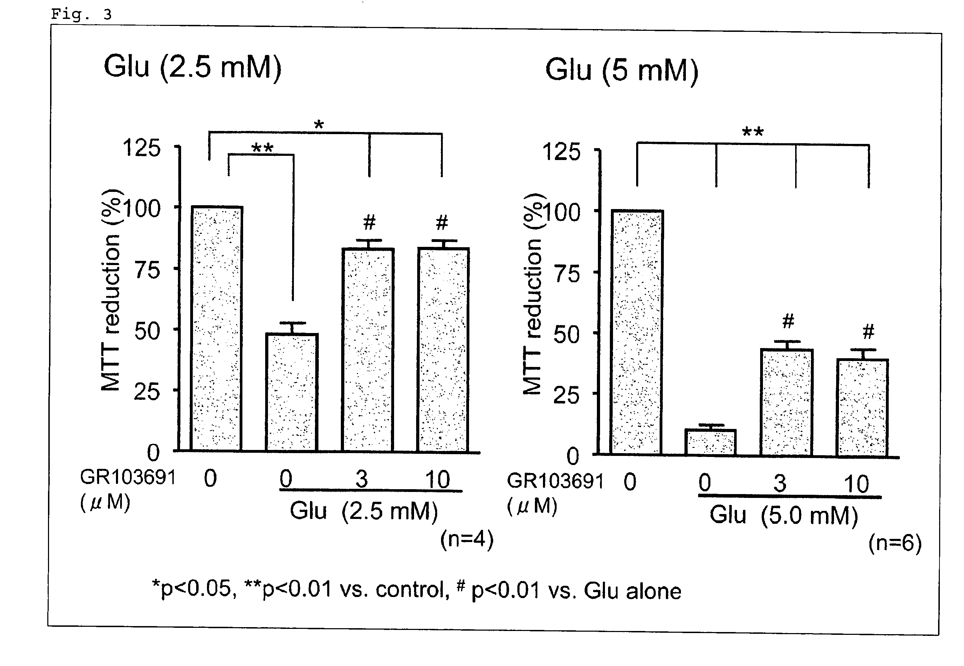

### (3) The influence of GR103691 on glutamic acid-induced cell death is shown in Figure 3.

MTT assay was conducted 24 hours after addition of glutamic acid. GR103691 was added immediately before the addition of glutamic acid (Glu).

The influence of GR103691 on glutamic acid-induced cell death was studied in order to examine whether or not the cell death inhibitory effect of GR103691 is specific for hypoxia/reoxygenation-induced cell death. MTT reduction caused by glutamic acid, i.e., cell death, was observed. However, GR103691 significantly recovered the reduction of MTT value caused by glutamic acid.

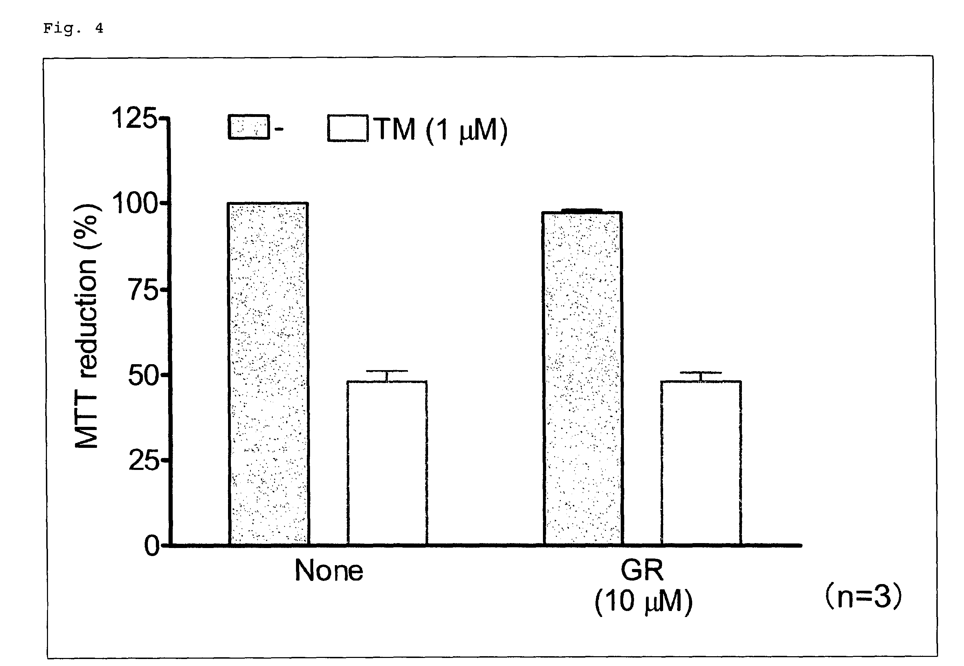

### (4) The influence of GR103691 on tunicamycin-induced cell death is shown in Figure 4.

MTT assay was conducted 24 hours after addition of tunicamycin (TM). GR103691 was added immediately before the addition of tunicamycin (TM).

TM-induced cell death is cell death induced by endoplasmic reticulum stress. MTT reduction caused by TM, i.e., cell death, was observed. GR103691 did not influence the reduction of MTT value caused by TM.

These results demonstrated that GR103691 has inhibitory effect on oxidative stress-induced apoptosis.

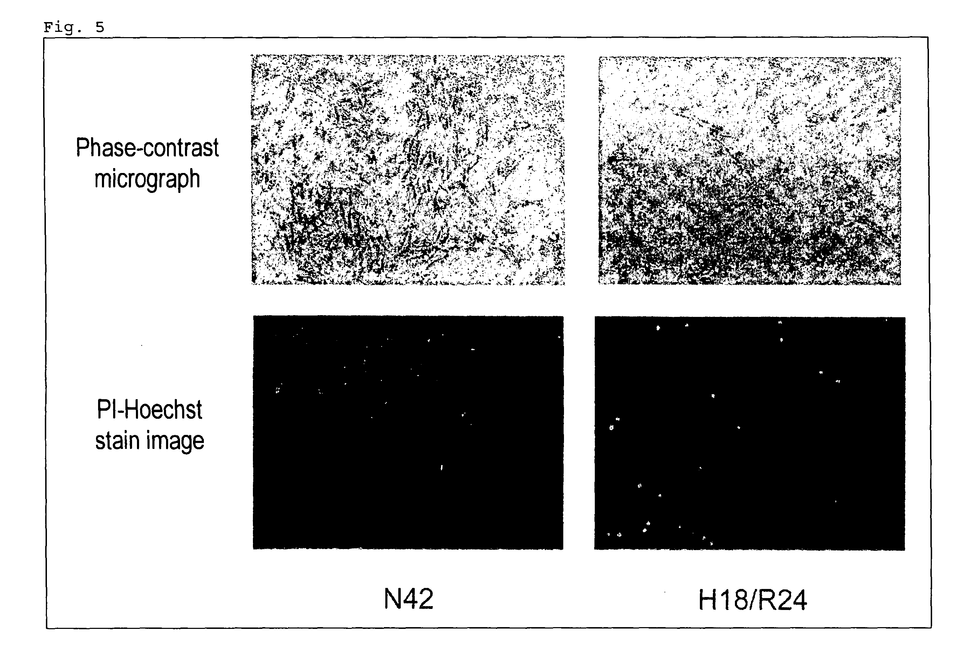

### (5) A PI stain image of hypoxia/reoxygenation-induced cell death is shown in Figure 5.

These images are micrographs of control and H18/R24-treated cells double-stained with Hoechst 33258 (Hoechst) and propidium iodide (PI).

Figure 5 shows micrographs of control or H18/R24-treated cells double-stained with Hoechst 33258 (Hoechst) that stains live cells blue and propidium iodide (PI) that stains dead cells red. All the control cells were stained with Hoechst but not with PI. The H18/R24-treated cells were smaller in number than the control, and PI-stained cells, i.e., dead cells, were observed among them. This cell observation demonstrates that hypoxia/reoxygenation-induced cell death is derived from apoptosis.

### Example 2

### (Method)

### (1) Cell culture

HT22 cells were cultured in DMEM containing 10% FBS in a 10% CO₂ incubator at 37°C.

### (2) Hypoxia-reoxygenation loading

Hypoxia loading was performed by using an oxygen absorber/CO₂ generator AnaeroPack Kenki For Cell^{™}, and reoxygenation was performed by restoring the condition to normoxia.

### (3) Intracellular reactive oxygen species (ROS) assay

Intracellular ROS levels were measured by using H₂DCFDA. Cells were treated as intended and then treated with 5 µM H₂DCFDA. After 15 minutes, the cells were observed under a fluorescence microscope (480 nm/526 nm).

### (Results)

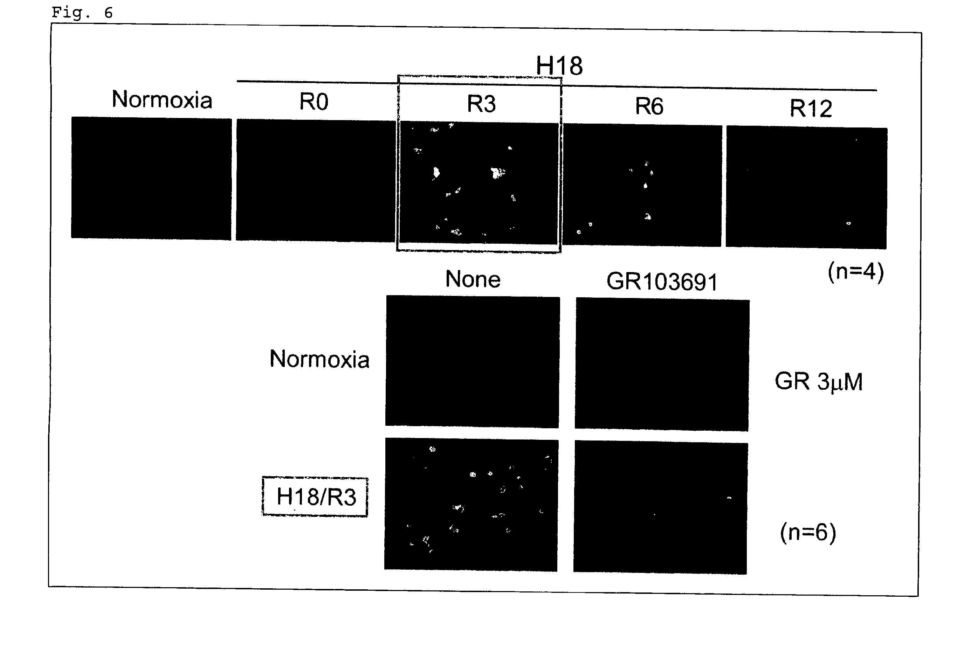

Time-dependent change of ROS was examined during reoxygenation for 12 hours after hypoxia for 18 hours (H18). Cells placed under normoxia for the same hours (N30) were used as a control. GR103691 was added at a concentration of 3 µM immediately before reoxygenation (or after 18-hour culture to the control cells).
The influence of GR103691 on increase of intracellular reactive oxygen species (ROS) caused by hypoxia-reoxygenation loading is shown in Figure 6. Figure 6 shows fluorescence micrographs of ROS stained with H₂DCFDA.

ROS emits fluorescence by H₂DCFDA treatment. H₂DCFDA-positive cells were not observed in the control cells at any point in time during the examination (Figure 6 shows micrographs after 30 hour-culture which is the longest culture time). By contrast, H₂DCFDA-positive cells were transiently observed by the reoxygenation treatment. The most H₂DCFDA-positive cells were observed after reoxygenation for 3 hours (H18R3). Then, the H₂DCFDA-positive cells decreased and were hardly observed after 12-hour reoxygenation. The transient increase of ROS observed in the H18/R3-treated cells was inhibited by the addition of GR103691.
Owing to the ROS increase inhibitory effect, GR103691 was presumed to impart inhibitory effect on oxidative stress-induced apoptosis such as hypoxia/reoxygenation-induced cell death and glutamic acid-induced cell death.

### Example 3

### (Method)

Male C57BL/6J mice (9-12 week old) were used. After halothane anesthesia, the left and right common carotid arteries were ligated for 17 minutes while the cerebral blood flow was monitored. Then, the blood flow was resumed to prepare a cerebral ischemia/reperfusion model. Paraffin sections of the brain were prepared 72 hours after reperfusion according to an ordinary method and immunostained with Fluoro Jade B (FJB) that stains dead cells green or with single-stranded DNA (ssDNA) that permits detection of apoptosis cells by the staining of nuclear fragmentation. The cells were observed under a microscope. GR103691 was administered at a dose of 1, 3, or 10 mg/kg from the tail vein of each mouse within 10 minutes after reperfusion. Moreover, for comparison, edaravone (manufactured by CALBIOCHEM) used for improving neurological symptoms, problems with movement of daily living, and dysfunction at the acute stage of brain infarction was administered at a dose of 3 or 10 mg/kg to each mouse. A saline-administered group and a sham group without only ischemia treatment were observed as controls.

### (Results)

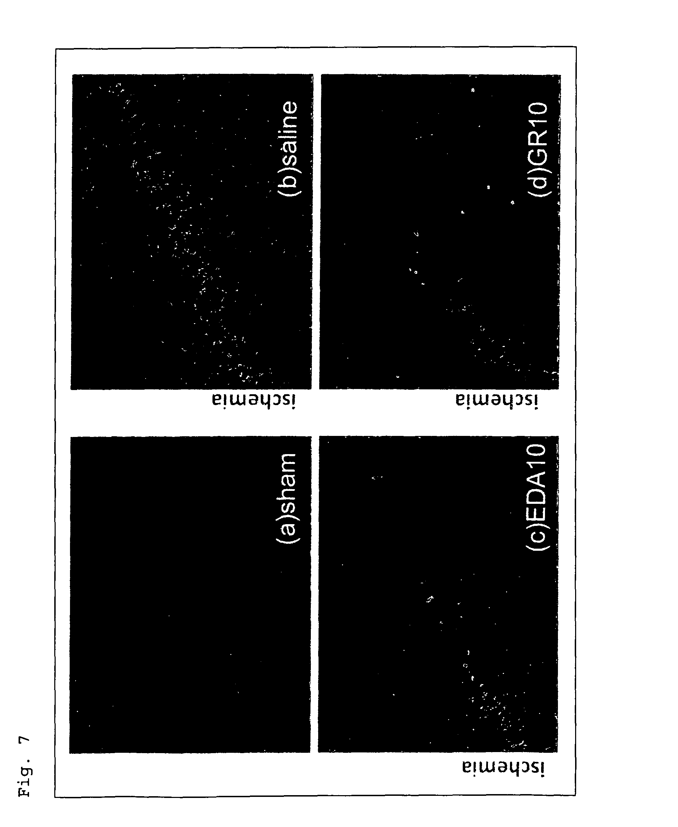

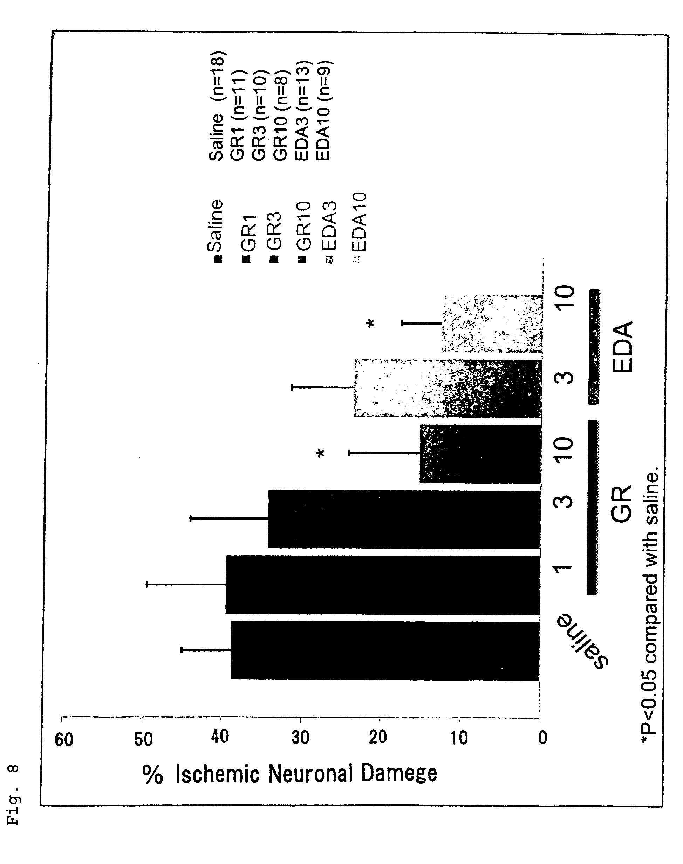

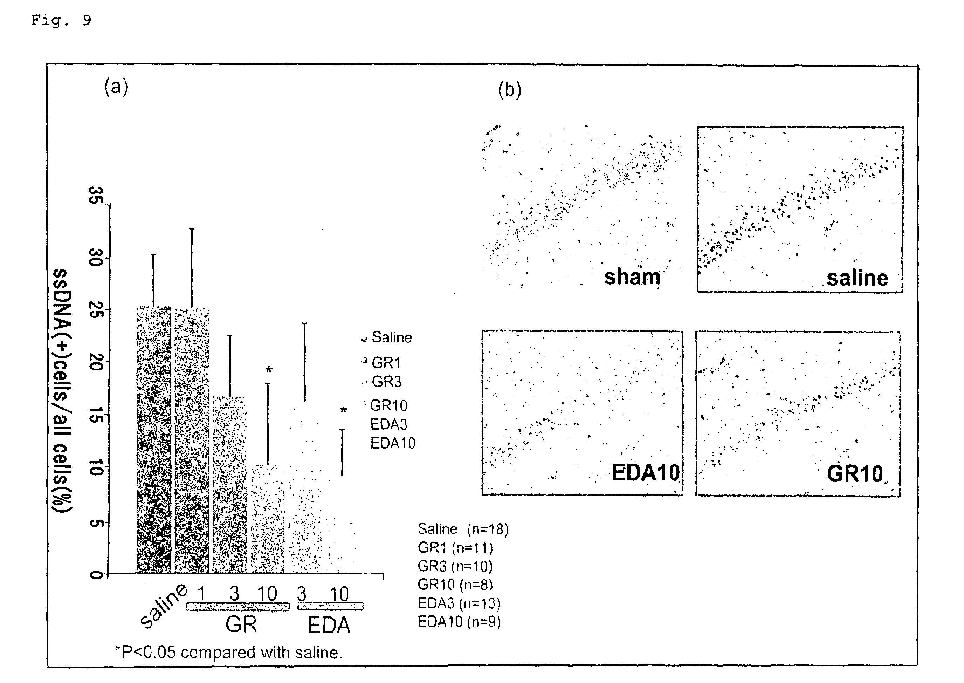

FJB stain images are shown in Figure 7. In Figure 7, the bright green color represents apoptosis cells. Moreover, the number of FJB-positive cells was counted to determine its proportion to the total number of cells (Figure 8). The results of each group are indicated as mean ± standard error.
In the cerebral ischemia/reperfusion model, cell death was observed in the hippocampal CA1 region vulnerable to ischemia-reperfusion injury (saline-administered group in Figure 7(b)). However, GR103691 inhibited such cell death in a dose-dependent manner. Particularly, the administration at a dose of 10 mg/kg significantly inhibited it. This inhibitory effect was almost the same level as that of edaravone (EDA) (Figure 8).
The proportion of ssDNA-positive cells to the total number of cells (Figure 9(a)) and ssDNA immunostain images (Figure 9(b)) are shown in Figure 9. In Figure 9(b), the pale purple color represents normal cells free from nuclear fragmentation, and the brown color represents apoptosis cells having nuclear fragmentation. The ssDNA-positive cells in the hippocampal CA1 region were decreased by GR103691 in a dose-dependent manner and significantly decreased particularly by the administration at a dose of 10 mg/kg. The decreasing effect was almost the same level as that of edaravone (EDA) (Figure 9).
These results demonstrated that GR103691 exerts inhibitory effect on oxidative stress-induced cell death not only in an *in-vitro* cultured cell system but also in an in-vivo system.

## Claims

1. An apoptosis inhibitor comprising, as an active ingredient, a biphenylcarboxamide compound represented by the following formula (1) : [Formula 1] 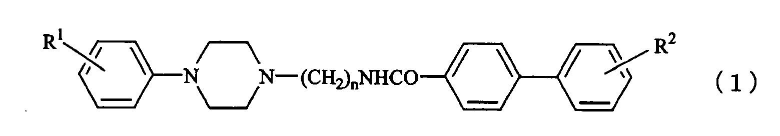 wherein R¹ represents a hydrogen atom or a lower alkoxy group; R² represents a hydrogen atom or a lower alkanoyl group; and n represents a number of 2 to 5.

2. The apoptosis inhibitor according to claim 1, wherein R¹ is a lower alkoxy group; R² is a lower alkanoyl group; and n is 4.

3. The apoptosis inhibitor according to claim 1, wherein R¹ is a methoxy group; R² is an acetyl group; and n is 4.

4. The apoptosis inhibitor according to any one of claims 1 to 3, wherein the apoptosis is an oxidative stress-induced apoptosis.

5. A preventive/therapeutic agent for a disease caused by an oxidative stress-induced apoptosis comprising, as an active ingredient, a biphenylcarboxamide compound represented by the following formula (1): 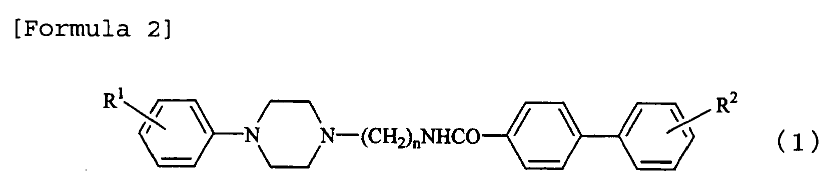 wherein R¹ represents a hydrogen atom or a lower alkoxy group; R² represents a hydrogen atom or a lower alkanoyl group; and n represents a number of 2 to 5.

6. The preventive/therapeutic agent according to claim 5, wherein R¹ is a lower alkoxy group; R² is a lower alkanoyl group; and n is 4.

7. The preventive/therapeutic agent according to claim 5, wherein R¹ is a methoxy group; R² is an acetyl group; and n is 4.

8. A pharmaceutical composition for apoptosis inhibition comprising a biphenylcarboxamide compound represented by the following formula (1) and a pharmaceutically acceptable carrier: 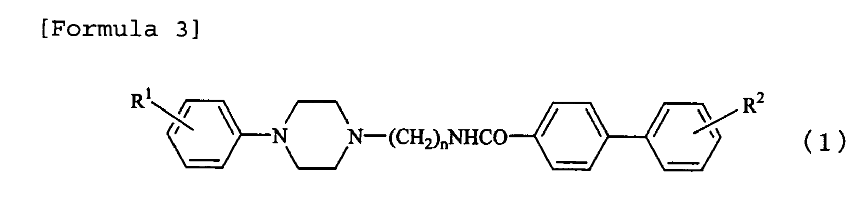 wherein R¹ represents a hydrogen atom or a lower alkoxy group; R² represents a hydrogen atom or a lower alkanoyl group; and n represents a number of 2 to 5.

9. The pharmaceutical composition for apoptosis inhibition according to claim 8, wherein R¹ is a lower alkoxy group; R² is a lower alkanoyl group; and n is 4.

10. The pharmaceutical composition for apoptosis inhibition according to claim 8, wherein R¹ is a methoxy group; R² is an acetyl group; and n is 4.

11. The pharmaceutical composition for apoptosis inhibition according to any one of claims 8 to 10, wherein the apoptosis is an oxidative stress-induced apoptosis.

12. A pharmaceutical composition for prevention/treatment of a disease caused by an oxidative stress-induced apoptosis, comprising a biphenylcarboxamide compound represented by the following formula (1) and a pharmaceutically acceptable carrier: 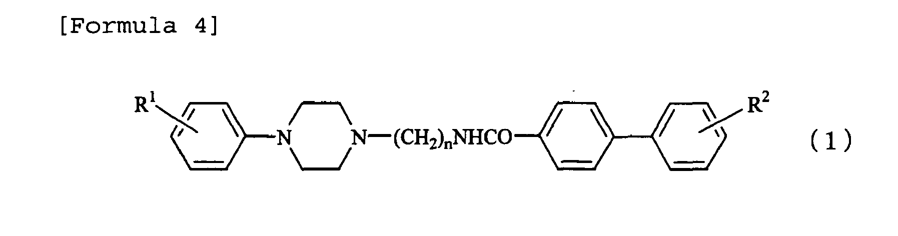 wherein R¹ represents a hydrogen atom or a lower alkoxy group; R² represents a hydrogen atom or a lower alkanoyl group; and n represents a number of 2 to 5.

13. The pharmaceutical composition for prevention/treatment according to claim 12, wherein R¹ is a lower alkoxy group; R² is a lower alkanoyl group; and n is 4.

14. The pharmaceutical composition for prevention/treatment according to claim 12, wherein R¹ is a methoxy group; R² is an acetyl group; and n is 4.

15. Use of a biphenylcarboxamide compound represented by the following formula (1) for production of an apoptosis inhibitor: 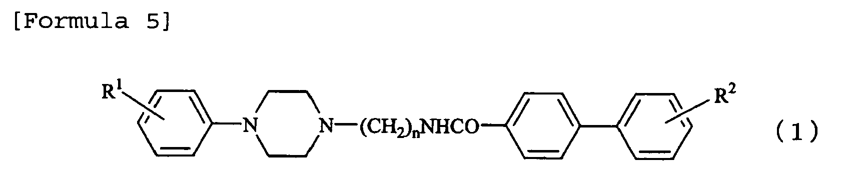 wherein R¹ represents a hydrogen atom or a lower alkoxy group; R² represents a hydrogen atom or a lower alkanoyl group; and n represents a number of 2 to 5.

16. The use according to claim 15, wherein R¹ is a lower alkoxy group; R² is a lower alkanoyl group; and n is 4.

17. The use according to claim 15, wherein R¹ is a methoxy group; R² is an acetyl group; and n is 4.

18. The use according to any one of claims 15 to 17, wherein the apoptosis is an oxidative stress-induced apoptosis.

19. Use of a biphenylcarboxamide compound represented by the following formula (1) for production of a preventive/therapeutic agent for a disease caused by an oxidative stress-induced apoptosis: 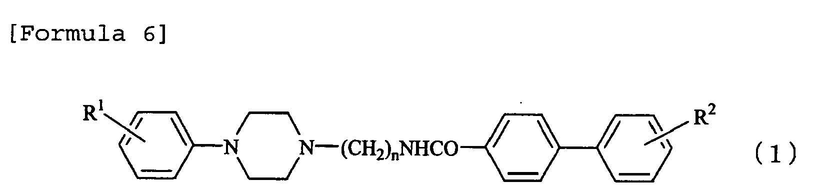 wherein R¹ represents a hydrogen atom or a lower alkoxy group; R² represents a hydrogen atom or a lower alkanoyl group; and n represents a number of 2 to 5.

20. The use according to claim 19, wherein R¹ is a lower alkoxy group; R² is a lower alkanoyl group; and n is 4.

21. The use according to claim 19, wherein R¹ is a methoxy group; R² is an acetyl group; and n is 4.

22. A method for inhibiting apoptosis, comprising administering an effective amount of a biphenylcarboxamide compound represented by the following formula (1): 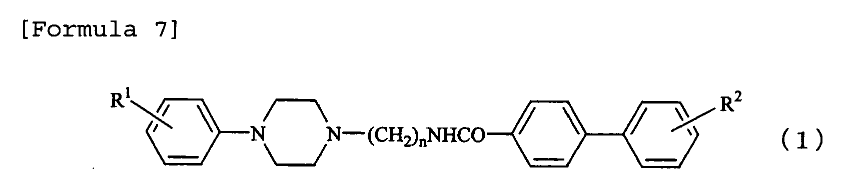 wherein R¹ represents a hydrogen atom or a lower alkoxy group; R² represents a hydrogen atom or a lower alkanoyl group; and n represents a number of 2 to 5.

23. The method for inhibiting apoptosis according to claim 22, wherein R¹ is a lower alkoxy group; R² is a lower alkanoyl group; and n is 4.

24. The method for inhibiting apoptosis according to claim 22, wherein R¹ is a methoxy group; R² is an acetyl group; and n is 4.

25. The method for inhibiting apoptosis according to any one of claims 22 to 24, wherein the apoptosis is an oxidative stress-induced apoptosis.

26. A method for preventing/treating a disease caused by an oxidative stress-induced apoptosis, comprising administering an effective amount of a biphenylcarboxamide compound represented by the following formula (1): 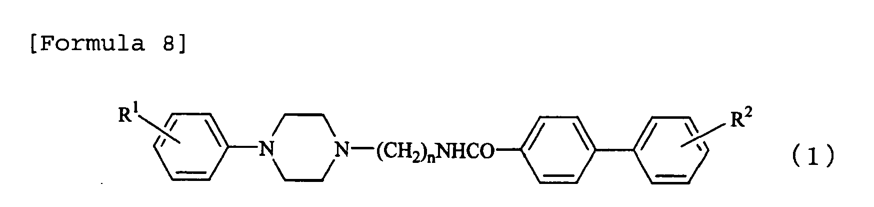 wherein R¹ represents a hydrogen atom or a lower alkoxy group; R² represents a hydrogen atom or a lower alkanoyl group; and n represents a number of 2 to 5.

27. The prevention/treatment method according to claim 26, wherein R¹ is a lower alkoxy group; R² is a lower alkanoyl group; and n is 4.

28. The prevention/treatment method according to claim 26, wherein R¹ is a methoxy group; R² is an acetyl group; and n is 4.
